# EUROPEAN PATENT APPLICATION

(11) **EP 1 790 732 A1**
(43) Date of publication of application: **30.05.2007**
(21) Application number: 06466012.9
(22) Date of filing: 23.08.2006
(51) Int. Cl.: C12P 3/00, C12P 5/02, C12P 7/00

(54) **Use of stillage from alcohol production**

(30) Priority: 24.08.2005 CZ 20050534
(71) Applicant: Prokop Invest, a.s., 533 01 Pardubice (CZ); Hydrotech s.r.o., 60200 Brno (CZ); Westfalia Separator CZ s.r.o., 10100 Praha 10 (CZ); CKD Praha DIZ a.s., 19002 Praha 9 (CZ)
(72) Inventor: Prochazka, Zdenek, 15200 Praha 5 Slivenec (CZ); Preisler, Jan, 12000 Praha 2 (CZ); Bocan, Peter, 83107 Bratislava - Vajnory (SK); Slaby, Frantisek, 53375 Dolni Redice 40 (CZ); Minovsky, Josef, 29477 Meceriz 74 (CZ)
(74) Representative: Vsetecka, Milos

(57) **Abstract**

The invention relates to a method of comprehensive use of stillage originating from bio alcohol mass production, while the end products of the process are biogas, dried stillage waste with low salt content, granulated sludge from anaerobic digestion, solid fertilizer on the basis of magnesium ammonium phosphate, elementary sulphur, sludge from aerobic final treatment, and waste heat from process water. According to the method, insoluble fraction is removed from the raw stillage by means of centrifugation, while remaining dispersed substances are removed from liquid fraction by air flotation, centrifugation, vacuum filtration, or a combination of these processes in the secondary separation step. Thickened dense fractions from both of these steps, having low salt content, are further processed to feed of high nutritional value. Liquid fractions from both of these steps are blended and acidified under controlled conditions at pH ranging between 4.8 and 9.2. Then, they arc taken to anaerobic digestion to the reactor with granulated biomass, wherein the said liquid fractions enter the reactor in its bottom part. The biomass consists of acidifying and methanogenic bacteria and the anaerobic digestion is performed at the temperature between 25 and 40°C, residence time 2 to 50 hours, and the granulated biomass loading rate between 5 to 105 COD/m⁻³.day⁻¹. The accumulated granulated sludge is removed and dried. Biogas exhausted from the anaerobic reactor head is biologically desulphurized, and elementary sulphur is produced as a by-product. The biogas is then used energetically. From the liquid fraction nitrogenous substances are removed by dosing magnesium chloride and phosphoric acid resulting in precipitation of insoluble magnesium ammonium phosphate that is separated and removed as a high-quality fertilizer. The liquid fraction is further taken to the aerobic final treatment where sludge is separated. After having been thickened, the sludge can be utilized in agriculture. From the aerobic final treatment plant treated water having parameters meeting requirements for release to watercourse is taken. It is then discharged or re-used in the process after an optional distillation. Furthermore, the invention relates to the device needed to perform the method of the invention.

## Description

### Field of the invention

The invention relates to a method of comprehensive use of stillage from bio alcohol mass production, while the end products of the process are biogas, dried stillage waste with low salt content, granulated sludge from anaerobic digestion, solid fertilizer on the basis of magnesium ammonium phosphate, elementary sulphur, sludge from aerobic final treatment, and waste heat from process water.

### Background of the invention

At present, the disposal of stillage from bio alcohol mass production plants represents a serious problem as the bio alcohol is produced in large amounts and will be produced in even larger amounts in the future as a renewable energy source added to vehicle fuels. In these mass production plants, stillage is produced in large amounts, and hence it is necessary to deal with its economical use, or find a method of convenient disposal thereof.

The stillage produced can be disposed in agricultural areas, which, however, results in the devastation of the areas due to the stillage high salt content. The salts are introduced into the stillage from raw material as well as during processing of the raw material for alcohol production. Furthermore, the stillage can be made thicker in evaporators, and the final product can either be utilized as cattle feed supplement, or can be burnt down. In both cases it is necessary to make the stillage thicker and dewater it, which is always technologically demanding. However, due to high salt content the thickened and dewatered stillage can be used as feed supplement to a limited extent only.

Some experiments with the stillage anaerobic digestion were conducted but the process faced some serious technological problems.

### Description of the invention

The above-mentioned shortcomings have been remedied by development of a method of comprehensive use of stillage from bio alcohol mass production according to the present invention. The invention relates to the removing insoluble fraction from raw stillage by means of centrifugation, while remaining dispersed substances are removed from the liquid fraction by air flotation, centrifugation, vacuum filtration, or a combination of these processes in secondary step of separation. Thickened dense fractions from both these steps, having low salt content, are further processed to feed of high nutritional value. Liquid fractions from both these steps are blended and acidified under controlled conditions at pH ranging between 4.8 and 9.2. The resulting mixture is then taken to anaerobic digestion carried out in the reactor with granulated biomass,wherein the mixture enters the reactor in its bottom part.. The said biomass consists of acidifying and methanogenic bacteria and the anaerobic digestion proceeds at the temperature between 25 and 40°C, residence time 2 to 50 hours, and the granulated biomass loading rate between 5 and 105 COD/m⁻³.day⁻¹. The accumulated granulated sludge is removed and stored for sale. Sulphur from biogas exhausted from the head of the anaerobic reactor is removed biologically, and elementary sulphur is produced as a by-product. The biogas is then exhausted to be used energetically. From the liquid fraction, nitrogenous substances are removed by dosing magnesium chloride and phosphoric acid resulting in precipitation of insoluble magnesium ammonium phosphate that is separated and removed as a high-quality fertilizer. The liquid fraction is subsequently taken to aerobic final treatment where sludge is separated. After having been thickened, the sludge can be used in agriculture. From the final treatment plant, treated water, having parameters meeting requirements for release into watercourses, is taken. It is then discharged, or re-used in the process after optional distillation.

The most important advantage of the process according to the invention is an utter utilization of energetic potential of stillage from bio alcohol production as biogas is produced from centrifuged stillage after applying anaerobic process to granulated anaerobic biomass. By means of centrifugation and subsequent drying of insoluble substances, cattle feed of high nutritional value is obtained that has considerably lower salt content in comparison to the conventional feed made of thickened stillage. The salts are discharged in a liquid stream at the centrifuge outlet. Drying the solid phase does not cause any problems as concerns burning on heat-exchanging surfaces, or carrying organic substances away in a stream of vapour. Following colloids and fine particles separation and after the acidification, anaerobic digestion process is applied to the liquid phase from the centrifuge. Following completion of the digestion process the liquid phase undergoes aerobic final treatment, after which the treated water will meet standards for its discharge in watercourses. Alternatively, the treated liquid phase can be recycled in the technological process itself. After desulphurisation, and parallel production of elementary sulphur, the biogas obtained can be used for energetic purposes either in the distillery production process, or for the production of electricity or vapour.

For dewatering corn stillage decantation centrifuges with a double gear drive are preferably used.The centrifuges are filled from a balance tank by means of pumps. A centrifuged cake is transported for drying by means of a conveyor. Syrup - i.e. liquid stillage - is discharged from the balance tank for second protein separation process wherein residual undissolved particles, especially cereal proteins, are separated so that protein content in the feed is higher. Revolutions of the centrifugal basket range between 1,000-10,000 min⁻¹, g-force ranging between 1,300-11,000 g. Differential revolutions range between 500 - 3,500 min⁻¹. The content of dry matter is between 20 - 40 % of the cake weight.

The process of final separation of suspended solids serves primarily for removing proteins, and hence increase the content of nitrogenous substances in the feed. Secondarily this process protects the anaerobic step from possible disintegration of granules by long-term action of suspended solids. Syrup from the first separation step is led to colloid separation process in the secondary separation step comprising air flotation, a centrifuge, or a vacuum filter with a sedimentary layer, or a combination thereof. For flotation, recirculation of treated water behind aerobic final treatment is utilized ranging between 5 - 100 % of the raw syrup inflow. At the same time, pre-precipitation of sulphates to calcium sulphate is carried out by dosing calcium oxide in the amount between 0.5 - 5 kg CaO/m³ of the liquid; in additionflocculant XA can be optionally added in the amount ranging between 0.01 - 0.1 kg/m³. Dry matter content of the cake obtained is 4 - 35 %.The treated liquid is then led in the acidification and balance tank.

The sludge obtained in the previous step is homogenized by stirring with a stirrer, and medium bubble aeration with the intensity of 0.5 - 10 m³m⁻³.h⁻¹, with the residence time in accumulation between 0.1 - 5 h. For dewatering the sludge, separating centrifuges or sludge presses can be used. The separating centrifuges are filled from the balance tank by means of pumps. The centrifuged cake having the dry matter content of 15 - 40 % is transported by means of a conveyor to drying process together with the cake from the first step. The liquid fraction is led to the acidification and balance tank together with the liquid from the previous steps.

The cakes and sludge from previous steps are preferably dried in a double rotor tubular dryer with recirculation of air substances and dried stuff. The wet cake and sludge are transported by a screw conveyor and a rotary volume dosing machine to a mixer wherein both of these input raw materials are mixed and homogenized. The pre-treated raw material is dosed from the mixer into the double rotor tubular dryer wherein water evaporates from the raw material under moderate vacuum to the required final moisture content of 10 % of the weight. Preferably, the dryer is a contact one.Saturated heating steam is used as a heating medium. The evaporated vapour, which has the temperature of 100°C, passes through a filter, an inert gas separator, a grease sedimentator, and a ventilator into an exchanger wherein the vapour condensate is utilized for heating air that is added to the dryer in order to reduce the heating steam consumption. Condensed water, which has the temperature of 90 - 95°C, is preferably used for the production of distilled water for production process making use of its heat content. The condensed water itself can be added to acidification. The dried product leaves the dryer on a screw conveyor, and 10 - 90 % is re-used in the process, while 90 - 10 % is transported to pelletizing. The dried stuff is transported through a permanent magnet to a grinder. Then, the dried stillage is pneumatically transported through a mixer to a cyclofilter. Air is forced from the cyclofilter to aspiration and to deodorization processes; dried stillage is sent through a sealing mechanism to a conditioner and a dosing screw conveyor that preferably forms a part of the conditioner. The dosing screw conveyor doses the dried stillage in an automatic ring granulator. From the granulator pellets fall in a granule cooler. Cooling air is blown in the granule cooler by a ventilator, and the polluted air from the granule cooler is treated in a filter, and is subsequently sent to aspiration and deodorization. The amount of dried stuff is 80 - 160 t.m⁻³ of dried stillage with the dry matter content of 90%.

Combined liquids from the preceding steps are led to acidification carried out under controlled conditions in the acidification tank with the residence time between 0.5 - 50 h , and with acidification percentage 3 - 86 % of acidifiable COD. pH in the acidification tank is maintained in the range between 4.8 and 9.2. In the acidification process, liquid is mixed by a stirrer, and the conditions are set so that the proportional content of fatty acids C₂-C₁₅ preserves sludge granulation in anaerobic reactor without facing any problems with granules disintegration. pH is adjusted by dosing alkalis and acids, especially by dosing NaOH and MgO amounting to 0.1 - 3 kg.m⁻³ of the liquid. Raw inlet water is cooled down from 70-80 °C by an air cooler, and diluted by diluting water, which is biologically treated, to the temperature of about 40°C. A portion of the effluent stream from the acidification tank, amounting to 0 - 15 % of the inflow, goes to anaerobic process for the purpose of denitrification processes.

Anaerobic digestion of the acidified liquid is carried out by means of granulated biomass in the system that is resistant to overload, and can cope with high concentrations of suspended solids in the inflow water. At the same time, it works under high load with the system of single and double separation of granules-gas-liquid mixture. The system is also self-regulating by recirculation forced by a biogas mammoth pump. Upward flow rate in the reactor ranges between 0.25 - 12 m.h⁻¹, the residence time is between 2 - 50 hours, sludge loading rate ranges between 5 - 105 COD m⁻³.day⁻¹, and the operating temperature ranges between 25 - 40 °C. The reactor is filled from a mix tank by pumps. Pre-treated liquid is gravitationally drained from the anaerobic reactor into the reactor for nitrogenous substances precipitation. pH in the mix tank is adjusted by dosing alkalis and acids, especially by dosing NaOH in the amount of 1- 50 kg.m⁻³ of the liquid. Anaerobic granulated sludge is produced in the amount of 0.01-0.05 kg.kg⁻¹ of the removed COD, and it is a saleable commodity. COD reduction is at least 90 %.

The next step is the precipitation of nitrogenous substances from the pre-treated water in the form of insoluble magnesium ammonium phosphate, which is Struvit - a commercial fertilizer of nutritionally high value. The aim is to maximally decrease nitrogen concentration flowing to the aerobic process so that the aerobic step is reduced to minimum. pH and ammonia content are adjusted by dosing MgCl₂ in the amount of 0.1 - 2 kg.m⁻³ of the liquid, and H₃PO₄ in the amount of 0 - 1 kg.m⁻³ of the liquid. Crystal salt is removed by an arc sieve, and is bagged as a saleable fertilizer. The amount of Struvit is 0.5-5 kg.m⁻³ of raw stillage.

The biogas produced in the anaerobic reactor has a high percentage of methane, up to 85 %.Prior to its use it is necessary to desulphurize the gas as the alcohol production process uses sulphuric acid, and sulphates transform from water to hydrogen sulphide in biogas. The biogas is preferably desulphurized as follows: a biogas stream is subject to biological desulphurisation resulting in production of elementary sulphur, which in turn can be further commercially utilized, or recycled and utilized for the production of sulphuric acid. The percentage of sulphur removed from the biogas is up to 98 %.The amount of sulphur is 0.4 - 4 kg.m⁻³ of raw stillage.

The desulphurized biogas is accumulated in a balancing gas tank, and is either burnt in cogeneration units with subsequent production of electricity, or in the distillery itself in a boiler house for the production of steam for the distillery energetic purposes. It is either added to the natural gas, or used as additive in other fuels. The sale of electrical energy to the electric network, or direct consumption thereof in the distillery is beneficial from the economical point of view. The equipment also comprises a burner of excessive biogas. The amount of biogas ranges between 15 -25 m³.m⁻³ of the raw stillage with possible production of electrical energy between 35 - 58 kWh.m⁻³ of raw stillage, and supplementary heat in the form of warm water of 80 - 90°C in the amount of 60 - 100 kWh.m⁻³ of raw stillage.

The subsequent aerobic digestion of water from the aerobic process treats only 2 - 10 % of the input pollution, including a portion of the raw effluent stream from the acidification process in the amount of 0 - 15% of the inflow going to aerobic process for the denitrification processes. The aerobic process itself comprises a first stage pre-denitrification process - DI, first stage nitrification process - NI, second stage denitrification process - DII, second stage nitrification process - NII, final settling tanks and regeneration of activated sludge - R. When abbreviated, the process is designated with R-D-NI-DII-NII symbols. The process brings effluent in the amount of 0 - 100 % of the effluent inflow into DI, and in the amount of 100 - 0 % of the effluent inflow into DII. The system comprises two inner recirculations going always from the end of a nitrification process into a denitrification process of the respective stage in the amount of 0 - 100 % of the inflow, and a large outer recirculation from the final settling tank - DN - to regeneration - R - in the amount of 10 - 200 % of the inflow. From the regeneration the sludge overflows and drains gravitationally into DI stage.

Following aerobic final treatment, the water flows into final settling tanks, wherein it has to meet parameters and Czech legal standards concerning treated effluent drained into a watercourse. In the aerobic final treatment process, excessive sludge is produced. Having been dewatered in centrifuges, or sludge presses, it is disposed by depositing in a landfill or by use in agriculture and gardening by composting, or by burning down in boiler houses as energetic fuel. The amount of sludge in the form of 20% dry matter reaches 0.05-0.1 m³.m⁻³ of raw stillage; for sludge dewatering, it is necessary to dose polyflocculant in the amount between 0.001 and 0.01 kg.kg⁻¹ of the sludge dry matter. Sludge loading rate in aerobic process is between 0.03 - 0.15 kg BOD₅.kg_{MLSS}⁻¹.day⁻¹ so that the process is capable of parallel denitrification and nitrification. For final precipitation of COD, phosphorus, and the colour, FeCl₃ is dosed in the amount of 0.1-10 kg.m⁻³ of raw stillage. For separating the sludge mixture in final settling tanks, scraper device is preferably used in longitudinally flown tanks with concurrent flow of sludge and water. For the separation of sludge from the sludge mixture, surface loading rate of 0.2 - 1.5 m.h⁻¹ and surface loading rate of MLSS 0.5 - 14 kg MLSS.m⁻².h⁻¹ can be applied while maintaining the parameter of residence time ranging between 0.5 - 10 h.

Aerobically treated water can be used in recirculation for acidification (20 - 80 % of raw water inflow) and flotation (80 - 20 % of raw water inflow), or possibly for replenishing cooling circuits. Preferably, the main recirculation is led into evaporators for distilled water production for the distillery production purposes; as a source of heat is used excessive heat from the distillery, that is applied to the evaporator under lower pressure. Evaporators can be sanitized in a CIP station; the sanitisation preparations used are disposed in the framework of aerobic step of effluent treatment.

A bio distillery with the consumption of 600t of wheat per day is eventually, in addition to the bio alcohol, capable of producing up to:
- 20,000 to 80,000 kWh/day of electrical energy,
- 80 to 160 t of dried stuff/day with the dry matter of 90 % of the weight as feed,
- 0.4 - 1.5 t of granulated sludge/day with the dry matter of 10 % for needs of waste water treatment plants,
- 0.5 - 2.5 t of Struvit/day (fertilizer MgNH₄PO₄),
- 0.4 - 2.0 t of elementary sulphur/day with purity up to 96 %,
- 40,000 - 160,000 kWh/day of heat in the form of warm water of 80 °C, and
- 5 - 20 t of aerobic sludge/day in the form of 20% dry matter.

| Parameters of treated water after aerobic final treatment range between: | | | | | |
|---|---|---|---|---|---|
| - | COD _{Cr} | 40 - 190 mg/l | - | BOD₅ | 5 - 25 mg/l |
| - | MLSS | 5 - 30 mg/l | - | N_{TOTAL} | 15 - 50 mg/l |
| - | SO₄ | 50 - 300 mg/l | - | DIS (dissolved inorganic salts) | 600 - 2,000 mg/l |
| - | P_{TOTAL} | 2 - 10 mg/l | - | T | 20 - 40 °C |

and it can be preferably use for recirculation in alcohol production by passing it through an evaporator, or directly for diluting raw stillage when being acidified.

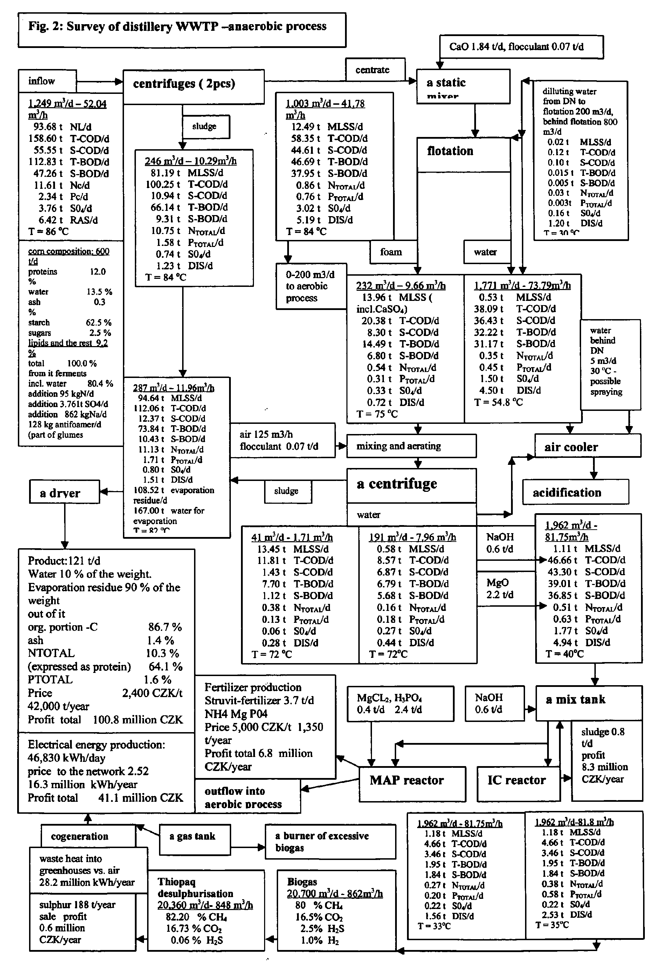

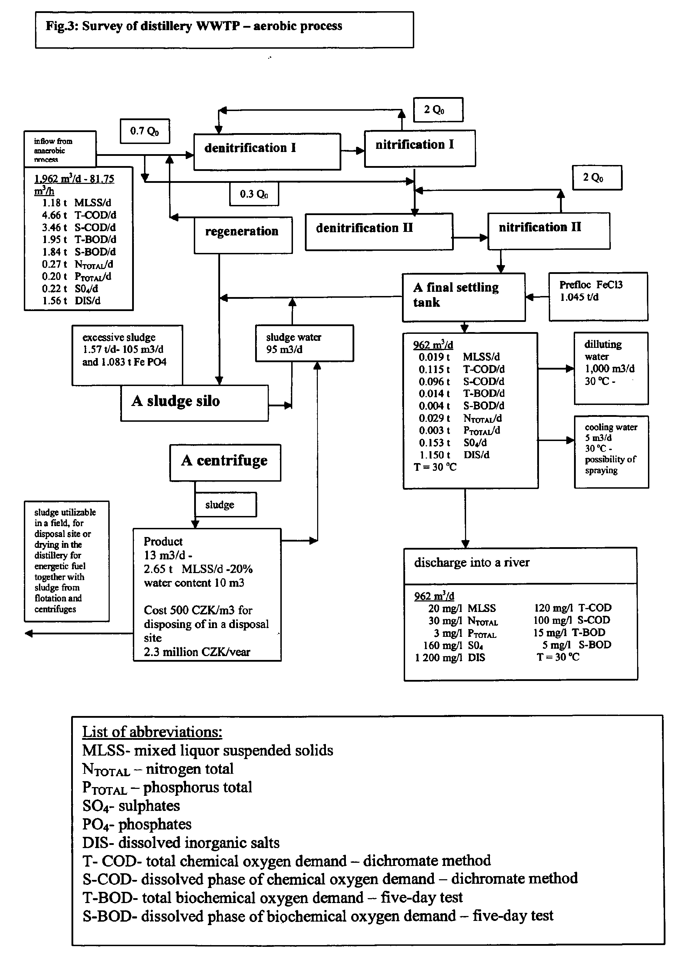

The present invention further relates to a device needed for the performance of the method of the invention, as shown in the attached Figure 1.

The device consists of a stillage storage tank 101 that is connected with decanting centrifuges 103-1,2 by a pipeline, and with a balance tank 106 A by a liquid phase pipeline. The balance tank 106 A is connected with an aerobic reactor 601 A,B,C,D,F by a water pipeline, and with an acidification tank 504 A by a water pipeline going through a stirring machine 503 B, a flotation final treatment unit 503 A, and a cooler 504 C. From the flotation final treatment unit 503 A, a pipeline with flotation sludge is led to a collecting tank, or to a centrifuge 508 that are connected with sludge reservoirs 104-1,2 by a pipeline. Downstream to the acidification tank 504 A, there is a mix tank 505 A, and an anaerobic reactor 506 A. The head of the reactor 506 A_is connected with a desulphurisation unit 701, and a gas tank 702 A by a biogas pipeline, and the anaerobic reactor 506 A is further connected with a MAP reactor 507 A, which is equipped with chemical dispensers 507 B-1,2, and a crystal separator 507 D by a water pipeline running through the mix tank 505 A. The bottom part of the anaerobic reactor 506 A is connected with a sludge tank 506 C by a pipeline discharging granulated anaerobic sludge produced. The MAP reactor 507 A is connected with the aerobic reactor 601 A,B,C,D,F with an air inlet 601 E by water pipeline. The air inlet 601 E is connected with a treated water tank 603 through a final settling tank 602 A. From the final settling tank 602 A, the sludge pipeline leads to the sludge tank 604 A, and a thickening unit 605 A. From the decanting centrifuges 103-1,2, the sludge pipeline leads to sludge storage tanks 104-1,2. From the tanks the pipeline leads either to a collecting tank 107, or to a dryer 204-1,2, where further processing is carried out.

### The above described device works as follows:

From the stillage storage tank 101 stillage is transported by means of pumps 102-1,2 to the decanting centrifuge 103-1,2. From the centrifuge 103-1,2 a stream of liquid phase is sent to the balance tank 106 A, from where portion of the water is drawn by a pump 106 B into the aerobic part of waste water treatment plant 601, while the main stream drains gravitationally to the secondary separation of suspended solids, including antifoaming oils 503 A. Part of aerobically treated water from the treated water tank 603, flocculant and calcium oxide is added to the secondary separation through the stirring device 503 B by dosing pumps 503 C-1,2,3. From the secondary separation unit, the liquid phase divested of the substantial part of suspended solids drains gravitationally to mixed acidification tank 504 A, where the remaining portion of recirculated treated water from the tank 603, magnesium oxide, and caustic soda is added by dosing pumps 504 B-1,2,3. A stream of secondarily separated suspended solids having a sufficient dry matter content of 30 % is either sent directly to a dosing scale of position 104-1,2 together with sludge from 103-1,2, or having an insufficient dry matter content, the stream is dewatered in a machine dewatering unit following air mixing, and organic starch coagulant addition. Said dewatering is preferably carried out in the centrifuge 508.

Prior to flowing to the acidification, water is cooled down in an air cooler 504 C to the temperature convenient for acidification processes, and the water acidified to a certain degree is then drawn by a pump 504 D into a mix tank 505 A equipped with a stirrer. A dosing pump 505 B doses caustic soda for pH adjustment into the mix tank 505 A, while the water in the mix tank is mixed with recirculated water from the anaerobic reactor in a T-device, and is then drawn with pumps 504 C into the anaerobic reactor 506 A. The anaerobic reactor 506 A cleans effluent using anaerobic biomass spontaneously granulated without any support, 1 - 20 separating stages of the sludge-gas-liquid mixture, and a biogas mammoth pump, and a separating and antifoaming head. At the same time, biogas and sellable excessive granulated biomass is produced and accumulated in a sludge tank 506 C for excessive anaerobic granulated sludge that is drawn into the tank by a pump 506 B.

Biogas from the top of the reactor is exhausted into a balancing gas tank 702 A through either a biological or chemical desulphurisation unit 701, wherein caustic soda is dosed, and elementary sulphur is produced which is then stored in a container. The biogas is then forced to cogeneration units or gas turbines 703 by means of a blower 702 B to produce electrical energy and waste heat, or to a boiler house as an auxiliary or main fuel for steam production. The biogas equipment also comprises a burner of excessive biogas 705, and a cooling system of cooling water from the production of electrical energy 704, unless the heat is used otherwise.

Water from the mix tank is drained gravitationally into the MAP reactor 507 A, wherein magnesium chloride and phosphoric acid is dosed by means f dosing pumps 507 B-1,2, and wherein air is forced by means of a blower 507 C so that precipitated magnesium ammonium phosphate forms crystals that are periodically removed from the MAP reactor using an arc sieve 507 D. The crystals are put in bags, and the stream of sieved liquid is pumped to the aerobic part 601 by a pump 507 E.

Tanks 504 A, 505 A, 506 A, 507 A are covered, and gas is drawn off into a deodorization filter 801 A for the air substance to be deodorized. The said gas is drawn off using a ventilator in Ex-variant 801 B-1,2,3,4.

Water from the MAP reactor drains gravitationally into aerobic part of the waste-water treatment plant. Larger fraction of the water is led to denitrification I 601 A, wherein it is mixed with a stirrer, and the mixture of activated sludge and water is drained gravitationally into an aerated nitrification I tank 601 B1, wherein it is aerated by blowers 601 E and aerating elements. The mixture is then sent to denitrification II 601 C, wherein it is mixed by a stirrer, and the mixture of activated sludge and water drains gravitationally into an aerated nitrification II tank 601 D1, wherein it is aerated by means of a blower 601 E and aerating elements. Then, the sludge mixture from the nitrification II goes into final settling tanks 602 A, from where the returned settled sludge is recirculated through the outer large recirculation circuit, using pumps 602 B, to the regeneration 601 F, wherein it is aerated by means of pressurised air from blowers 601 E and by means of aerating elements. For both nitrification stages, sludge mixture is internally recirculated from the end of a nitrification tank into the denitrification tank of the respective stage by pumps 601 B2, 601 D2, and at the same time ferric chloride and flocculant is dosed into the inflow of final settling tanks 602 A by pumps 601 B-1,2 in order to precipitate excessive phosphorus and dye, and to reduce COD. The treated water drains gravitationally through overflow edges of final settling tanks into the treated water tank 603. From the tank 603 it is drawn with pumps 504 B-1, 504 C-1 to the acidification tank 504 A and into the stirring machine 503 B.

The excessive sludge from the aerobic part system of the waste-water treatment plant is drawn periodically into a sludge tank 604 A by means of pumps 604 B. When replenishing the tank with further sludge, the sludge water is automatically gravitationally drained from the tank. At that time the aeration of aerating system of aerobic sludge stabilisation and blowers 604 D are turned off. The water returns via a pipeline into the regeneration tank 601 F. Having been dosed with flocculant by a flocculation station and polyflocculant pumps 604 E, and dewatered in the centrifuge 605 A with the final dry matter content between 20 - 30 %, the sludge thickened to approx. 3 % of dry matter is drawn by means of pumps 604 C so that it can be alternatively drawn with pumps 605 B and dried together with sludge from decanting centrifuges 103 - 1,2, and from secondary separation of suspended solids 503 A. In case the sludge mixture is not sold for feeding purposes, it would then serve as energetic fuel for steam production, or the aerobic sludge can be stored after dewatering, or composted.

Sludge and cakes are transported gravitationally or by means of pumps into stirred and weighed storage tanks 104-1,2, from where the mixed sludge is dosed by a dosing volume and piston device 105-1,2 into the mixer 203-1,2 wherein at the same time a portion of dried mixture is added through recirculation 220-1,2. Homogenized stream of up to three kinds of raw material and cakes is sent into the double rotor tubular dryer 204-1,2 with the recirculation of air substance and dried stuff, where steam from the plant boiler house and recirculated air from the dryer is forced. The recirculated air is warmed in the air exchanger 212-1,2 by saturated heating steam. For recirculation, a high-pressure ventilator 211-1,2 is used, while evaporated vapour from the dryer having the temperature of 100°C passes through a vapour filter 205-1,2 into a water vapour condenser 208-1,2. From there inert gases pass through an inert gas separator 209-1,2 into the above-mentioned ventilator 211-1,2. Water from the vapour condenser 208-1,2 flows off into a grease interceptor 210-1,2, and can be further used in the distillery production process. The dried stuff from the double rotor tubular dryer 204-1,2 is transported on a discharging screw conveyor 206-1,2 and a cranked bulk conveyor 207-1,2 through an aspiration attachment 207 A-1,2 for separating dust back upstream the mixer feeding 203-1,2. From there the non-recirculated dried stuff is transported on screw conveyors 201-1,2 and 213-1,2 to dried stuff processing prior to expedition.

The dried stuff from the above-mentioned conveyors passes through a permanent magnet 301-1,2 to separate metal objects from the dried stuff stream, and passes into a vertical grinder 301-1,2, and into a mixer of pneumatic transportation 303-1,2, and a cyclofilter 304-1,2, where the dried stuff is separated from air, which is released into the atmosphere. The dried stuff subsequently drops gravitationally through a sealing machine 305-1,2 into a mixer and a conditioner 306-1,2 and a granulating press 307-1,2, and then into a granule cooler 308-1,2. Air is utilized as coolant. The air is forced into the cooler by a ventilator 309-1,2, and the air from the cooler passes through a filter with deodorization 310-1,2 so that taking fine particles out in the atmosphere is avoided.

Granulated dried stuff is transported by a bucket elevator 401 into a system of direct bulk conveyers 402, 404 with aspiration attachments 403,405, and into steel silos 406-1,2,3. Then, it is dropped through a vibrating bottom 407-1,2,3 gravitationally on a direct feeding bulk conveyer 408 with an aspiration attachment 410. From the bulk conveyer, the product is transferred via telescopic nozzles 409-1,2 into shipping containers for bulk material, in which it is transported to final use destination, or a disposal site.

### Example

The invention is further described with reference to the below illustrative example presented as a block scheme showing flow rates and the composition of individual streams.

## Claims

1. A method of comprehensive use of stillage from bio alcohol mass production,
**characterized in that** insoluble fraction is removed from raw stillage by centrifugation, while remaining dispersed substances are removed from liquid fraction by means of air flotation, centrifugation, vacuum filtration, or a combination thereof in the secondary separation stage; thickened dense fractions from both of these steps, having low salt content, are further processed to feed of high nutritional value; the liquid fractions from both the of these steps are blended and acidified under controlled conditions at pH ranging between 4.8 and 9.2 and subsequently taken to anaerobic digestion to the reactor with granulated biomass, wherein the said liquid fractions enter the reactor in its bottom part, the biomass consists of acidifying and methanogenic bacteria, and the anaerobic digestion is carried out at the temperature between 25 and 40°C, residence time 2 to 50 hours, and granulated biomass loading rate between 5 and 105 COD/m⁻³.day⁻¹; the accumulated granulated sludge is removed and dried and biogas drawn off from the anaerobic reactor head is biologically desulphurized with production of elementary sulphur as a by-product; the biogas is then drawn off to be used energetically; from the liquid fraction nitrogenous substances are removed by dosing magnesium chloride and phosphoric acid resulting in precipitation of insoluble magnesium ammonium phosphate that is separated and removed as a high-quality fertilizer and the liquid fraction is further taken to the aerobic final treatment where sludge is separated, that, after having been thickened, can be utilized in agriculture; following the sludge separation the treated water having parameters meeting requirements for release into watercourse is taken is further taken from the final treatment plant, and this water is then discharged or re-used in the process after an optional distillation.

2. The method according to claim 1,
**characterized in that** the insoluble fraction is removed by decantation centrifugation between 1,000 and 10,000 min⁻¹, and at force ranging between 1,300 and 11,000 g, and the centrifuged cake is dewatered.

3. The method according to claim 1,
**characterized in that** colloids, especially proteins, are secondarily separated from the liquid fraction by air flotation after the separation of insoluble substances, and at the same time pre-precipitation of sulphates present is carried out by dosing calcium oxide and flocculant and the obtained sludge is dewatered.

4. The method according to claim 1,
**characterized in that** sludges obtained according to claims 2 and 3 are mixed, dewatered by centrifugation, or sludge presses, and dried.

5. The method according to claim 1,
**characterized in that** the liquid obtained after the separation of solid fractions is acidified under controlled conditions at residence time ranging between 0.5 and 50 h, at pH between 4.8 and 9.2 with the degree of acidification between 3 to 86 % of acidifiableCOD, while caustic soda and magnesium oxide are being dosed.

6. The method according to claim 1,
**characterized in that** the anaerobic decomposition of acidified liquid is performed in a reactor by means of granulated biomass comprising acidifying and methanogenic bacteria, spontaneously multiplied and granulated without a support at the temperature ranging between 25 and 40 °C and at upward flow rate of the liquid in the reactor between 2 and 12 m.h⁻¹.

7. The method according to claim 1,
**characterized in that** nitrogenous substances precipitate in the anaerobically treated water by adding magnesium chloride in the amount of 0.1 to 2 kg.m⁻³, and phosphoric acid in the amount of 0 to 1 kg.m⁻³, and precipitated magnesium ammonium phosphate is separated.

8. The method according to claim 1,
**characterized in that** biogas released from the anaerobic digestion process is biologically desulphurized in order to remove the hydrogen sulphide and produce elementary sulphur.

9. The method according to claim 1,
**characterized in that** the final treatment of the liquid fraction obtained from the anaerobic digestion and precipitation of magnesium ammonium phosphate is carried out by anaerobic digestion with the excessive sludge being dewatered, thickened and removed to be utilized in agriculture or burnt down.

10. A device to perform the method according to claim 1,
**characterized in that** it consists of a stillage storage tank (101) that is connected with decanting centrifuges (103-1,2) by a pipeline, and with a balance tank (106 A) by a liquid phase pipeline; the balance tank is connected with an aerobic reactor (601 A,B,C,D,F) by a water pipeline, and with an acidification tank (504 A) by a water pipeline going through a stirring machine (503 B), a flotation final treatment unit (503 A), and a cooler (504 C); from the flotation final treatment unit (503 A), a pipeline with flotation sludge is led to a collecting tank, or to a centrifuge (508) that are connected with sludge reservoirs (104-1,2) by a pipeline; downstream to the acidification tank (504 A), there is a mix tank (505 A), and an anaerobic reactor (506 A) which head is connected with a desulphurisation unit (701), and a gas tank (702 A) by a biogas pipeline, and the anaerobic reactor (506 A) is further connected with a MAP reactor (507 A), which is equipped with chemical dispensers (507 B-1,2), and a crystal separator (507 D), by a water pipeline running through the mix tank (505 A); the bottom part of the anaerobic reactor (506 A) is connected with a sludge tank (506 C) by a pipeline discharging the produced granulated anaerobic sludge; the MAP reactor (507 A) is connected with the aerobic reactor (601 A,B,C,D,F) with an air inlet (601 E) by a water pipeline and the said air inlet is connected with a treated water tank (603) through a final settling tank (602 A); from the final settling tank (602 A) the sludge pipeline is led to the sludge tank (604 A), and a thickening unit (605 A), and from the decanting centrifuges (103-1,2), the sludge pipeline leads to sludge storage tanks (104-1,2), from the sludge storage tanks (104-1,2) the pipeline leads either to a collecting tank (107), or to a dryer (204-1,2) where the dried stuff are further processed.
